# EUROPEAN PATENT APPLICATION

(11) **EP 1 749 747 A1**
(43) Date of publication of application: **07.02.2007**
(21) Application number: 05107171.0
(22) Date of filing: 03.08.2005
(51) Int. Cl.: B65B 55/02, B67C 7/00, B67B 3/06, B65G 47/84

(54) **Cap processing device**

(71) Applicant: ASEPTIS, S.L., 08210 Barberà del Valles (ES)
(72) Inventor: Quetin, Arnaud, 08037 Barcelona (ES); Fonte, Josep, 08192 Sant Quirze del Vallès-Barcelone (ES)
(74) Representative: Barlocci, Anna

(57) **Abstract**

The device (1) is intended for processing caps (2) of the type comprising a closure portion (2a) and a body portion (2b). The device (1) comprises a number of rotating cylinders (3a, 4a, 4b, 5a, 5b) for engaging and transporting the caps (2) during cap processing. There is provided at least a first rotating cylinder (3a; 4b; 5b) with first engagement means (6) for engaging the cap body portion (2b) and at least a second rotating cylinder (4a; 5a) having second engagement means (7) for engaging the cap closure portion (2a). Cylinders (3a, 4a, 4b, 5a, 5b) are arranged such that caps (2) may be transferred from one cylinder to another. Some of the cylinders (4a, 4b) may be cylinders in a sterilising section (4) for effectively sterilising the caps (2).

## Description

The present invention refers to a cap processing device for processing caps which is specially intended for processing caps of the type comprising a closure portion and a body portion or spout. The closure portion acts a cap and the body portion is intended to be inserted within a bag like package.

The invention also refers to a method of sterilising caps and a packaging machine including such a cap processing device.

### BACKGROUND ART

Cap processing devices are known in the art comprising a series of gear wheels carrying caps which are transferred from one gear wheel to another gear wheel and transported through sections for cleaning or sterilising the caps.

One example of such devices is disclosed in JP11193009 that refers to a cap processing device comprising main gear wheels and auxiliary gear wheels, all of them carrying caps. The auxiliary gear wheels are provided between some of the main gear wheels so that at least two main gear wheels may be rotated in the same direction. Gear wheels are arranged such that the peripheries thereof are adjacent to one another and they are vertically rotated inside a clean room divided into a sterilising section, in which caps are sterilised by using spraying water to the caps; a washing section, in which caps are washed by steamy water; and a drying section in which caps are dried by injecting sterile air to the caps. Pipes provided with openings are provided inside the sections for injecting water and air to the caps.

JP11193009 is intended for processing standard cylindrical caps by applying sterilising water to each cap. This requires the use of at least three pipes provided with respective openings: one pipe for the cap lateral surface, another pipe for the cap outer surface and a further pipe for the cap inner surface. No provision is made for another type of caps other than cylindrical caps and water injection does not seem to ensure an efficient sterilisation.

### SUMMARY OF THE INVENTION

The present invention provides a device which it has been found that is capable of effectively processing caps of the type comprising a closure portion and a body portion. Said processing may be, for example, sterilising. As it will be in the following, the invention allows effectively sterilising the closure portion and the body portion of such caps.

The device comprises a number of rotating cylinders intended to engage and transport the caps during cap processing. Said cylinders may be arranged rotating around a horizontal axis inside sections in the device.

More particularly, at least a first rotating cylinder is provided with first engagement means for engaging the cap body portion and at least a second rotating cylinder is provided with second engagement means for engaging the closure body portion. The cylinders are arranged such that caps may be transferred from one cylinder to another. This permits an effective cap processing as the caps are passing from cylinder to cylinder.

As outlined above, the device of the present invention may be used for sterilising caps. In this case, first and second rotating cylinders are provided in a sterilising section for engaging and transport the caps during sterilisation thereof. The first and second cylinders in the device are rotatably mounted inside the sterilising section which is under a sterilising atmosphere at a negative pressure, for example a sterilising medium such as hydrogen peroxide in gas phase. The sterilising section may be located between a heating section and a drying section.

In some embodiments, some of said sterilising, heating and drying sections are connected to each other by one way valve means through which caps are allowed to be passed for transferring said cap from one cylinder in one section to another cylinder of an adjacent section.

Preferably, the device of the invention comprises vacuum applying means for holding the caps in position in the cylinders as the cylinders are rotated. Said vacuum applying means operate in conjunction with a rotating valve system which allows said vacuum applying means to be driven in a predetermined angular position of the cylinder. Caps are held by said vacuum applying means through a part of their travel, the rest of their travel being held by upper and lower support guides.

There may be also provided air blowing means for blowing air to the caps for assisting them to be discharged out of the device through a duct in which caps advance in a single position. Devices are impelled by the air and they fall by gravity to a turner assembly where each cap is flipped over in such a way that the cap closure portion in disposed in an upper position. As the cap is flipped over, the body portion becomes inserted into the package.

The device of the invention may be provided with a cap transferring device intended for forcing a cap to pass from one cylinder to another cylinder. Said cap transferring device may comprise an assembly comprising arms connected to a common supporting member which is reciprocated in a synchronous manner with cylinder rotation so that one cap is pushed by one arm out of the cylinder where it is received causing it to be received in an adjacent cylinder. Alternative embodiments of such cap transferring device may comprise a plunger acting synchronously with rotation of the cylinders pushing one cap out of the cylinder where it is received causing it to be received to the adjacent cylinder.

In another embodiment of the device of the present invention, on the one hand, the first engagement means are peripheral recesses formed on the surface of each of the cylinders. Said peripheral recesses have an inner profile that is complementarily shaped to the outer profile of the cap body portion. On the other hand, the second engagement means are peripheral recesses formed on the surface of the cylinder. Said peripheral recesses have an inner profile complementary to the outer profile of said cap closure portion.

The peripheral recesses in the first and second engagement means may be shaped such that they prevent the cap from being rotated relative to the recess where it is received. This ensures a proper positioning of the caps relative to the packages when inserting the body portion of the caps into the packages.

The peripheral recesses in said first and second engagement means may be selectively connected to the vacuum applying means so that caps are held in place in the respective recesses as necessary.

A method of sterilising caps is also provided with the present invention which is intended to be used for sterilising the above describe type of caps, that is, those comprising a closure portion and a body portion. Said method of sterilising caps comprises the following steps:
- feeding the caps into a rotating cylinder for heating the caps with hot sterile air;
- transferring the caps from said rotating cylinder to a first rotating cylinder in a sterilising section for exposing one of a cap closure portion and cap body portion under a sterilising atmosphere;
- transferring the caps from said first rotating cylinder to a second rotating cylinder in said sterilising section for exposing the other of said cap closure portion or cap body portion under the sterilising atmosphere;
- transferring the caps from said second rotating cylinder in the sterilising section to a first rotating cylinder in a drying section for exposing one of said cap closure portion and cap body portion under a drying atmosphere; and
transferring the caps from said first rotating cylinder in the drying section to a second rotating cylinder in said drying section for exposing the other of said cap closure portion or cap body portion under the drying atmosphere for removing any waste products from the sterilising process.

A packaging machine is also provided comprising a cap processing device as described above.

### BRIEF DESCRIPTION OF THE DRAWINGS

A particular embodiment of a cap processing device according to the present invention will be described in the following, only by way of non-limiting example, with reference to the appended drawings, in which:
Fig. 1 is a general side view of a cap processing device, specially used for sterilising caps, according to the invention;
Fig. 2 is an enlarged side view showing the various sections of the cap processing device in fig. 1; and
Fig. 3 is a detail part view of two adjacent cylinders carrying caps in the cap processing device of the invention.

### DETAILED DESCRIPTION OF PARTICULAR EMBODIMENTS

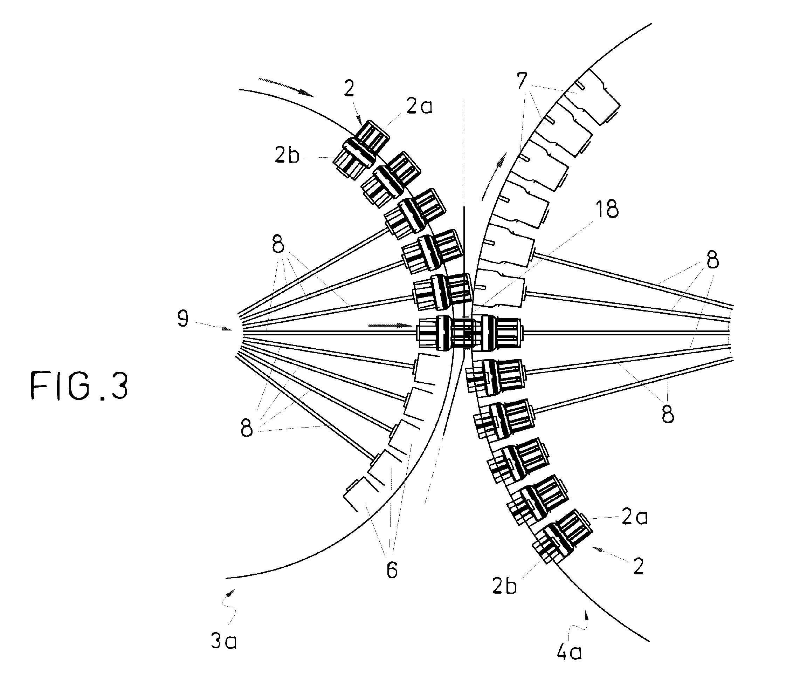

The device 1 of the example shown in the figures is a cap sterilising device used for sterilising caps 2. Caps 2 may be clearly seen in figure 3 where two cylinders 3a, 4a are partly shown. The caps 2 comprise a closure portion 2a and a body portion 2b. The closure portion 2a acts a cap and the body portion 2b is intended to be inserted within a bag like package (not shown).

The cap sterilising device 1 in the embodiment shown comprises a heating section 3, a sterilising section 4 and a drying section 5. A number of respective cylinders 3a, 4a, 4b, 5a, 5b are rotatably mounted inside sections 3, 4 and 5 around respective horizontal shafts for engaging and transporting the caps 2 during cap sterilisation.

In the embodiment of the figures, the cylinders 3a, 4a, 4b, 5a, 5b are adjacent to one another, but other intermediate elements between the cylinders 3a, 4a, 4b, 5a, 5b could be provided if necessary. Caps 2 are transferred from cylinder to cylinder within said sections 3, 4, 5 as the caps 2 are flipped over. For this purpose, alternate cylinders 3a, 4b and 5b are provided with first engagement means 6 for engaging the cap body portion 2b, as it may be seen from figure 3 of the drawings. The rest of the cylinders, that is, cylinders 4a and 5a, are provided with second engagement means 7 for engaging the cap closure portion 2a.

The first engagement means 6 and the second engagement means 7 will be fully described further on.

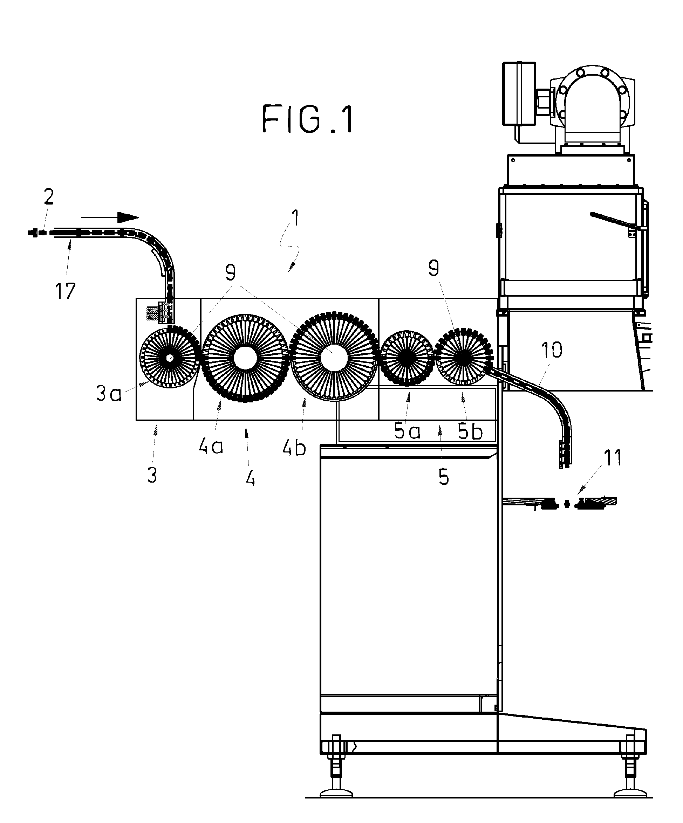

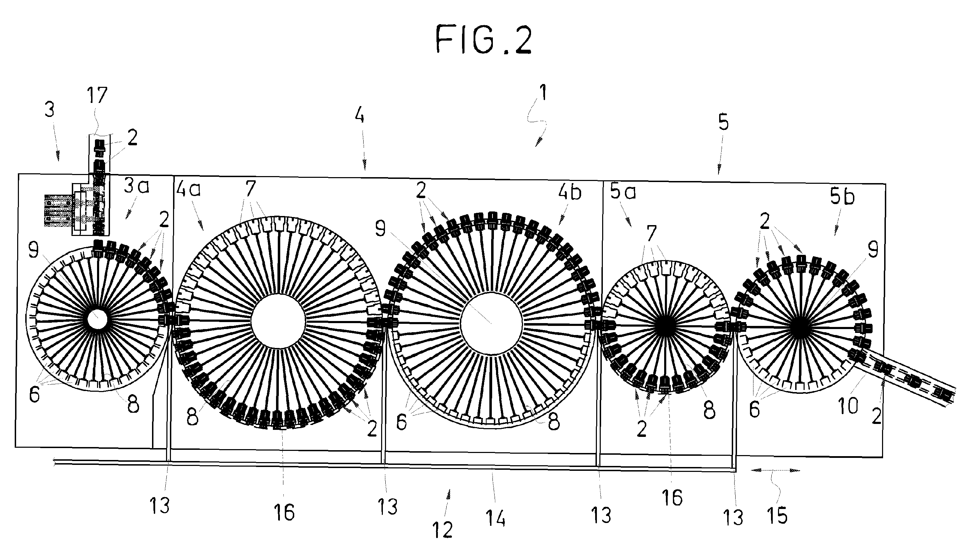

As shown in the figures 1 and 2, a cylinder 3a is rotatably mounted inside the heating section 3, two cylinders 4a, 4b are rotatably mounted inside the sterilising section 4, and two cylinders 5a, 5b are rotatably mounted inside the drying section 5.

The cylinder 5b in the drying section 5 communicates with a discharge tube 10 through which caps 2 are advanced in a single position. Output of the caps 2 from the device 1 is assisted by air blowing means (not shown). Caps 2 then fall by gravity out of the device 1 to a turner assembly 11 where each cap 2 is flipped over in such a way that the cap 2 becomes in an upright position, i.e. with the cap closure portion 2a disposed in an upper position. As the cap 2 is flipped over, the body portion 2b becomes inserted into the package (not shown).

The cylinders 3a, 4a, 4b, 5a, 5b are rotated in a reversed direction relative to one another for transferring the caps 2 in the above described manner, that is, by being flipped over so that both the closure portion 2a and the body portion 2b are effectively sterilised.

The sections 3, 4, 5 are isolated from one another since different atmospheres are established therein. The sterilising section 4 may be at a negative pressure and under a sterilising atmosphere by using, for example, hydrogen peroxide. The diameters of the cylinders 3a, 4a, 4b, 5a, 5b depend on the cap exposure time in each corresponding section 3, 4, 5. For example, in the embodiment shown, the diameter of the cylinders 4a, 4b in the sterilising section 4 are larger than that of the cylinders 3a, 5a, 5b in the heating and drying sections, 3 and 5 respectively, since the sterilising process may take a longer time than heating or drying processes.

The heating, sterilising and drying sections 3, 4, 5 are connected to each other by one way valve means 18, see figure 3. Through said one way valve means 18, each cap 2 is allowed to be passed from one cylinder in one section to another cylinder of an adjacent section.

The caps 2 are retained in position in the respective recesses, while the cylinders are rotated, by means of vacuum applying means. The vacuum applying means, which are fitted at least in cylinders 3a, 4b, 5b, (in all the cylinders 3a, 4a, 4b, 5a, 5b in the embodiment shown) are operated in conjunction with a rotating valve system 9 which drives the vacuum applying means when the cylinders are in a predetermined angular position. The vacuum applying means include a series of ducts 8 leading to the engagement means 6, 7.

Caps 2 are held by said vacuum applying means through a part of their travel, the rest of their travel being held by support guides 16 which will be described further on.

The caps 2 are transferred from one cylinder to another cylinder through a cap transferring device 12 which may comprise, for example, an assembly having a series of arms 13 having a push member. The arms 13 are connected to a common supporting member 14 which is reciprocated in a synchronous manner (see arrow 15 in figure 2) with cylinder rotation. As the assembly with the arms 13 and the supporting member 14 is moved to the right in the figure 2, the end of the push members of said arms pushes one cap 2 out of the cylinder where it is received causing it to be received in the adjacent cylinder. Other cap transferring devices are possible, for example, comprising plungers acting synchronously with rotation of the cylinders pushing one cap 2 out of the cylinder transferring it to the adjacent cylinder.

As stated above, some of the cylinders have first engagement means 6 for engaging the cap body portion 2b while others have second engagement means 7 for engaging the cap closure portion 2a. The engagement means 6, 7 are peripheral recesses formed on the surface of the cylinders. In the case of the first engagement means 6, the recesses have an inner profile that is complementarily shaped to the outer profile of the cap body portion 2b while in the case of the second engagement means 7, the recesses have an inner profile that is complementarily shaped to the outer profile of the cap closure portion 2a.

The peripheral recesses in the first and second engagement means 6, 7 are shaped such that the cap 2 is prevented from being rotated relative to the recess where it is received. This ensures the cap 2 will be in a proper position when leaving the device 1 for being inserted into the package (not shown).

The peripheral recesses of said first and second engagement means 6, 7 may be selectively connected to the vacuum applying means through the above mentioned ducts 8.

In those cylinders 4a, 5a in which caps 2 are carried by the lower half of the cylinder (see fig. 2), lower support guides 16 are provided for preventing caps 2 from falling down from said cylinders 4a, 5a.

The method of sterilising caps in the above described device 1 is performed as follows (reference is made to figure 2 which clearly shows the different sections in the device 1 of the embodiment):
- caps 2 are fed through a feeding duct 17 into the rotating cylinder 3a of the heating section 3. The caps 2 present in the right, upper quarter of the cylinder 3a in heating section 3 are retained in position by their body portion 2b (see also figure 3) in the respective recesses of the first engaging means 6 by the effect of the vacuum applying means while the cylinder 3a is rotated clockwise such that caps 2 are heated with hot sterile air for being prepared for the sterilising process in the next section of the device 1;
- caps 2 are transferred, by means of the cap transferring device 12, from said cylinder 3a in the heating section 3 to the first cylinder 4a in the sterilising section 4 in such a way that the closure portion 2a of the caps 2 becomes inserted into the corresponding recess (second engaging means 7) of said first cylinder 4a in the sterilising section 4. Therefore, the body portion 2b of the caps 2 are sterilised since they are exposed to the sterilising medium in the sterilising section 4 as the first cylinder 4a is rotated counterclockwise. There is provided a lower support guide 16 arranged from the portion of the cylinder where the vacuum applying means do not act, as stated below, for preventing caps 2 located at its lower half, from falling down;
- caps 2 are transferred by the cap transferring device 12 from the first cylinder 4a to the second, adjacent cylinder 4b in the sterilising section 4. The caps 2 present in the upper half of said cylinder 4b are retained in position by their body portion 2b in the respective recesses of the first engaging means 6 by the effect of the vacuum applying means while said second cylinder 4b is again rotated clockwise. The closure portion 2a of the caps 2 is now sterilised as being exposed to the sterilising medium in the sterilising section 4 (there would be also provided an upper support guide which is arranged from the portion of the cylinder where the vacuum applying means do not act);
- caps 2 are transferred from the second cylinder 4b in the sterilising section 4 to the first rotating cylinder 5a in the drying section 5 so that the closure portion 2a of the caps 2 becomes inserted into the corresponding recess (second engaging means 7) of said first cylinder 5a in the drying section 5. Therefore, the body portion 2b of the caps 2 are dried since they are exposed to a drying medium in the drying section 5 as the first cylinder 5a in the drying section 5 is rotated counterclockwise. As with the first cylinder 4a in the sterilising section 4, a lower support guide 16 is provided arranged from the portion of the cylinder where the vacuum applying means do not act, for preventing caps 2 located at its lower half, from falling down;
- caps 2 are finally transferred from the said cylinder 5a in the drying section 5 to a second cylinder 5b in said drying section 5. Here, the caps 2 present in the upper half of the second cylinder 5b in the drying section 5 are retained in position by their body portion 2b in the respective recesses of the first engaging means 6 by the effect of the vacuum applying means while said second cylinder 5b in the drying section 5 is again rotated clockwise. The closure portion 2a of the caps 2 is now dried as being exposed to the drying medium in the drying section 5 for removing any waste products from the sterilising process. In this case, again, an upper support guide arranged from the portion of the cylinder where the vacuum applying means do not act would be also provided;
- caps 2 reaching the final length of the upper half in said second cylinder 5b in the drying section 5 are discharged out of the cylinder 5b in a single position by means of blowing means (not shown) through a discharge tube 10. Caps 2 then fall by gravity to a turner assembly 11 where each cap 2 is flipped over in such a way that its closure portion 2a is disposed in an upper position and its body portion 2b is inserted into a package (not shown).

The aforedescribed steps of the method performed by the device 1 are carried out synchronously and continuously.

It will be understood that although the method herein described according to one embodiment of the invention retains the cap first by their body portion 2b and then by their closure portion 2a in the next cylinder, the caps 2 could be suitably processed in the reverse order, that is, first by their closure portion 2a and then by their body portion 2b.

On the other hand, the device 1 may be of course fitted in a packaging machine having other sections or stations such as at least some of the following: a package feeding station, cutting and welding stations, a film sterilising station, a filling station, etc.

Although the device 1 of the example shown in the figures is intended for sterilising caps, it will be understood that the device of the present invention may be used for may other applications other than sterilising, such as for example, washing, etc.

## Claims

1. Cap processing device, said caps (2) comprising a closure portion (2a) and a body portion (2b), the device (1) comprising a number of rotating cylinders (3a, 4a, 4b, 5a, 5b) intended to engage and transport the caps (2) during cap processing, **characterised in that** at least a first rotating cylinder (3a; 4b; 5b) is provided with first engagement means (6) for engaging the cap body portion (2b) and at least a second rotating cylinder (4a; 5a) is provided with second engagement means (7) for engaging the cap closure portion (2a), said cylinders (3a, 4a, 4b, 5a, 5b) being arranged such that caps (2) may be transferred from one cylinder to another.

2. Cap processing device as claimed in claim 1, wherein said first and second rotating cylinders (4a, 4b) are intended to engage and transport the caps (2) during sterilisation thereof, said first and second cylinders (4a, 4b) being rotatably mounted inside a sterilising section (4).

3. Cap processing device as claimed in any of the preceding claims, wherein it further comprises vacuum applying means for holding the caps (2) in position in the cylinders (3a, 4a, 4b, 5a, 5b) as the cylinders (3a, 4a, 4b, 5a, 5b) are rotated, and a rotating valve system (9) for allowing said vacuum applying means to be driven in a predetermined angular position of the cylinder.

4. Cap processing device as claimed in any of the preceding claims, wherein it further includes air blowing means for blowing air to the caps (2) for assisting them to leave the device (1).

5. Cap processing device as claimed in any of claims 2 to 4, wherein it further comprises a heating section (3) and a drying section (5).

6. Cap processing device as claimed in claim 5, wherein sections (3, 4, 5) are connected to each other by one way valve means (18) through which caps (2) are allowed to be passed for transferring the cap (2) from one cylinder in one section to another cylinder of an adjacent section.

7. Cap processing device as claimed in any of the preceding claims, wherein it further includes a cap transferring device (12) for forcing a cap (2) to pass from one cylinder to another cylinder.

8. Cap processing device as claimed in claim 7, wherein said cap transferring device comprises arms (13) connected to a common supporting member (14) which is reciprocated in a synchronous manner with cylinder rotation so that one cap (2) is pushed by one arm (13) out of the cylinder where it is received causing it to be received in an adjacent cylinder.

9. Cap processing device as claimed in claim 7, wherein said cap transferring device (12) comprises a plunger acting synchronously with cylinder rotation pushing one cap (2) out of the cylinder where it is received causing it to be received to an adjacent cylinder.

10. Cap processing device as claimed in any of the preceding claims, wherein said first engagement means (6) are peripheral recesses formed on the surface of the cylinder (3a, 4b, 5b) having an inner profile complementary to the outer profile of said cap body portion (2b), and said second engagement means (7) are peripheral recesses formed on the surface of the cylinder (4a, 5a) having an inner profile complementary to the outer profile of said cap closure portion (2a).

11. Cap processing device as claimed in claim 10, wherein the peripheral recesses in the first and second engagement means (6, 7) are shaped such that the cap (2) is prevented from being rotated relative to the recess where it is received.

12. Cap processing device as claimed in claim 10 or claim 11, wherein the peripheral recesses in said first and second engagement means (6, 7) may be selectively connected to the vacuum applying means.

13. Cap processing device as claimed in any of the preceding claims, wherein said sterilising medium is hydrogen peroxide in gas phase.

14. Method of sterilising caps, the caps comprising a closure portion and a body portion, **characterised in that** it comprises the steps of:
- feeding caps (2) to a rotating cylinder (3a) for heating the caps (2);
- transferring the caps (2) from said cylinder (3a) to a first rotating cylinder (4a) in a sterilising section (4) for exposing one of a cap closure portion (2a) and cap body portion (2b) under a sterilising medium;
- transferring the caps (2) from said first rotating cylinder (4a) in the sterilising section (4) to a second rotating cylinder (4b) in said sterilising section (4) for exposing the other of said cap closure portion (2a) or cap body portion (2b) under the sterilising medium;
- transferring the caps (2) from said second rotating cylinder (4b) in the sterilising section (4) to a first rotating cylinder (5a) in a drying section (5) for exposing one of said cap closure portion (2a) and cap body portion (2b) under a drying medium; and
- transferring the caps (2) from said first rotating cylinder (5a) to a second rotating cylinder (5b) in said drying section (5) for exposing the other of said cap closure portion (2a) or cap body portion (2b) under the drying medium.

15. Packaging machine comprising a cap processing device (1) as claimed in any of claims 1 to 13.
